# EUROPÄISCHE PATENTSCHRIFT

(11) **EP 1 345 875 B1**
(45) Veröffentlichungstag und Bekanntmachung des Hinweises auf die Patenterteilung: **20.04.2005**
(21) Anmeldenummer: 01989550.7
(22) Anmeldetag: 28.11.2001
(51) Int. Cl.: C07C 29/20, C07C 35/08, C11B 9/00

(54) **VERFAHREN ZUR HERSTELLUNG VON CIS-2-TERTIÄR BUTYLCYCLOHEXANOL DURCH KATALYTISCHE HYDRIERUNG VON 2-TERTIÄR-BUTYLPHENOL**
METHOD FOR PRODUCING CIS-2-TERT-BUTYLCYCLOHEXANOL BY CATALYTICALLY HYDROGENATING 2-TERT-BUTYLPHENOL
PROCEDE POUR PRODUIRE DU CIS-2-TERT-BUTYLCYCLOHEXANOL PAR HYDROGENATION CATALYTIQUE DE 2-TERT-BUTYLPHENOL

(30) Priorität: 11.12.2000 DE 10061540
(43) Veröffentlichungstag der Anmeldung: 24.09.2003
(73) Patentinhaber: Symrise GmbH & Co. KG, 37603 Holzminden (DE)
(72) Erfinder: KUHN, Walter, 37603 Holzminden (DE)
(74) Vertreter: Eisenführ, Speiser & Partner
(86) Internationale Anmeldenummer: PCT/EP2001/014049
(87) Internationale Veröffentlichungsnummer: WO 2002/048079

(56) Entgegenhaltungen:
- H.L. GOERING, ET AL.: "The synthesis, assignment of configuration and dehydration of cis- and trans-2-t-butylcyclohexanol" JOURNAL OF THE AMERICAN CHEMICAL SOCIETY, Bd. 78, Nr. 19, 5. Oktober 1956 (1956-10-05), Seiten 4926-4931, XP002196172 American Chemical Society, Washington, DC, US ISSN: 0002-7863
- DATABASE WPI Section Ch, Week 197435 Derwent Publications Ltd., London, GB; Class E15, AN 1974-62050V XP002196196 & JP 49 045037 A (KAWAKEN FINE CHEMICALS), 27. April 1974 (1974-04-27) in der Anmeldung erwähnt
- B. SILBEROVA, ET AL.: "Hydrogenation of 2-tert-butylphenol over Ni catalyst" REACTION KINETICS AND CATALYSIS LETTERS, Bd. 67, Nr. 1, 1999, Seiten 29-33, XP001073518 Elsevier Science Publishers, Amsterdam, NL ISSN: 0133-1736

## Beschreibung

Die Erfindung betrifft ein Verfahren zur Herstellung von cis-2-tertiär Butylcyclohexanol aus 2-tertiär-Butylphenol, wobei unter den Hydrierbedingungen bevorzugt das cis-Isomer gebildet wird. 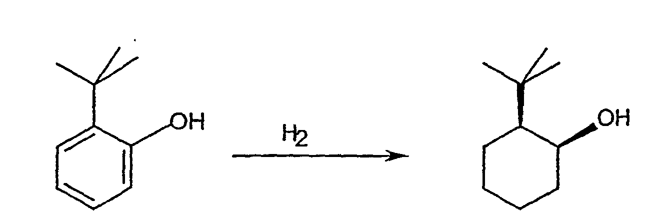 2-tertiär-Butylcyclohexanol, das in Form der cis und trans Stereoisomeren auftreten kann, ist ein wertvolles Zwischenprodukt für die Herstellung des Duftstoffes 2-tertiär-Butylcylohexylacetat. Bei der technischen Produktion von 2-tertiär-Butylcyclohexanol wird angestrebt große Anteile des cis-Isomers zu erhalten, um im Anschluss durch Veresterung das parfümistisch wertvolle cis 2-tertiär-Butylcyclohexylacetat zu erhalten, das unter dem Trivialnamen Agrumex HC bekannt ist.

Für die Hydrierung von 2-tertiär-Butylphenol zu 2-tertiär-Butylcyclohexanol sind Edelmetallkatalysatoren wie metallisches Rhodium, Rhodium-Platin- und Rhodium-Ruthenium-Legierungen bekannt, die auf Katalysatorträgern niedergeschlagen sind. (JP-A 42-13 938). Nachteilig bei der Hydrierung in Gegenwart eines Rhodiumkatalysators ist neben dem hohen Preis für den Katalysator die nur geringe Stereoselektivität im Hinblick auf das cis-Isomer und die Aktivitätsabnahme des Katalysators bei höheren Temperaturen. Daneben entsteht bei der rhodiumkatalysierten Hydrierung tertiär-Butylbenzol.

In DE A 3401343 wird die Hydrierung von 2-tertiär-Butylphenol zu 2-tertiär-Butylcyclohexanol in Gegenwart von Palladium und Ruthenium beschrieben, wobei in zwei Stufen bei Wasserstoffdrücken über 200 bar und Temperaturen von 70°-200°C gearbeitet wird. In der ersten Stufe setzt man einen Palladiumkatalysator und in der zweiten Stufe einen Rutheniumkatalysator ein. Das cis/trans-Verhältnis beträgt dabei bis zu 90/10.

In JP A 59/065031 wird die Hydrierung von 2-tertiär-Butylphenol zu 2-tertiär-Butylcyclohexanol unter Raney-Cobalt-Katalyse bei 50 bar und 150°C beschrieben, wobei das cis:trans-Isomerengemisch im Verhältnis 94:6 gebildet wird.

In DE A 2909663 wird die Hydrierung von 2-tertiär-Butylphenol zu 2-tertiär-Butylcyclohexanol unter Ruthenium-Katalyse bei 40 bar und 100°C beschrieben, wobei das cis:trans-Isomerengemisch im Verhältnis 92.5:7.5 gebildet wird.

In JP A 49/045037 wird die Hydrierung von 2-tertiär-Butylphenol zu 2-tertiär-Butylcyclohexanol unter Raney-Nickel-Katalyse bei 80 bar und 85°C beschrieben. Der Raney-Nickel-Katalysator wird vor Einsatz mit wässriger Natriumboranat-Lösung behandelt. Mit diesem so behandelten Katalysator wird das cis:trans-Isomerengemisch im Verhältnis 92:8 gebildet. Ohne Behandlung des Raney-Nickel-Katalysators entsteht das cis:trans-Isomerengemisch im Verhältnis 80:20.

Nachteilig bei dem beschriebenen Stand der Technik ist, dass die Standzeiten der Katalysatoren und der Anteil des cis-Isomeren nicht ausreichend sind.

Aufgabe der vorliegenden Erfindung ist es demgemäß, ein Verfahren zur Verfügung zu stellen, das preiswert 2-tertiär-Butylcyclohexanol mit hohem cis-Isomeranteil liefert und eine gegenüber dem Stand der Technik verlängerte Standzeit des Katalysators garantiert.

Es wurde ein Verfahren zur Herstellung von cis-2-tertiär-Butylcyclohexanol gefunden, das dadurch gekennzeichnet ist, dass 2-tertiär-Butylphenol in Gegenwart eines Nickel/Eisen Katalysatorgemisches und 2-tertiär-Butylcyclohexylacetat hydriert wird.

Erfindungsgemäß besonders bevorzugt ist der Einsatz von Raney-Nickel-Eisen-Katalysatoren. Die Verwendung dieser Katalysatoren in Kombination mit 2-tertiär-Butylcyclohexylacetat führt zu einem 2-tertiär-Butylcyclohexanol mit einem cistrans-Isomerengemisch von bis zu 95:5.

Mit dem Zusatz von 2-tertiär-Butylcyclohexylacetat kann die Standzeit des Katalysators erheblich verlängert werden. Es hat sich gezeigt, dass der Raney-Katalysator bei Zusatz des 2-tertiär-Butylcyclohexylacetats mehr als 10mal eingesetzt werden kann, ohne dass das cis-trans-Verhältnis unter 90:10 abfällt.

Vergleichsversuche haben dagegen gezeigt, dass ohne den Zusatz von 2-tertiär-Butylcyclohexylactat bei der Hydrierung das cis-trans-Verhältnis nur bei 92:8 liegt. Bei wiederholtem Einsatz des Katalysators sinkt das cis-trans-Verhältnis sogar noch weiter ab. Schon bei der dritten Wiederholung des Katalysatoreinsatzes ohne Zusatz von 2-tertiär-Butylcyclohexylacetat beträgt das Verhältnis nur noch 90:10.

Für das erfindungsgemäße Verfahren kann der Katalysator im trockenen oder feuchten Zustand verwendet werden.

Für das erfindungsgemäße Verfahren kann im trockenen Katalysator der Anteil von Eisen 2-40 Gew.-% bevorzugt 10-20 Gew.-%; der Anteil von Nickel 60-95 Gew.-% bevorzugt 70-85 Gew.-%, der Anteil von Aluminium 1-20 Gew.-% bevorzugt 3-10 Gew.-%, betragen.

Für das erfindungsgemäße Verfahren beträgt das Gewichtsverhältnis des eingesetzten Katalysators zu 2-tertiär-Butylphenol 0,0001 bis 0,1, bevorzugt 0,01 bis 0,03 zu 1.

Das Gewichtsverhältnis der Einsatzstoffe 2-tertiär-Butylphenol und 2-tertiär Butylcyclohexylacetat kann 100-0.2:1, bevorzugt 7-9:1 betragen.

Die Reaktionstemperatur für das erfindungsgemäße Verfahren liegt bei 50 bis 200°C, bevorzugt bei 90 bis 130°C.

Der Wasserstoffdruck liegt bei 1 bis 100 bar, bevorzugt bei 10 bis 20 bar.

Die Reaktionszeit beträgt 2 bis 100 Stunden, bevorzugt 5 bis 20 Stunden.

Das erfindungsgemäße Verfahren wird im allgemeinen wie folgt durchgeführt: In einem Druckbehälter mit Rührer werden 2-tertiär-Butylphenol, 2-tertiär Butylcyclohexylacetat und der Katalysator vorgelegt. Es wird bei der gewählten Reaktionstemperatur und Wasserstoffdruck hydriert. Das so erhaltene cis-2-tertiär Butylcyclohexanol wird nach Entfernen des Katalysators durch Filtration, Dekantieren oder Zentrifugation erhalten.

Das so erhaltene Rohgemisch kann ohne weitere Vorbehandlung mit Essigsäureanhydrid zum Zielprodukt cis-2-tertiär Butylcyclohexylacetat umgesetzt werden. Cis-2-tertiär Butylcyclohexylacetat ist ein Riechstoff mit holzig-fruchtigen Eigenschaften (S. Steffen Arctander, Perfume and Flavour Chemicals, No. 438, Montclair, New Jersey, 1969).

### Beispiele

### Beispiel 1

In einem Rührautoklaven mit Begasungsrührer werden 520 g 2-tertiär-Butylphenol, 80 g 2-tertiär Butylcyclohexylacetat und 17 g Raney-Nickel-Eisen (Wassergehalt 44 %, Nickelgehalt 45 %, Eisengehalt 8 %, Aluminiumgehalt 3 %) vorgelegt. Es wird 10 Stunden bei 130°C und anschließend 3h bei 100°C hydriert. Der Wasserstoffdruck beträgt 20 bar. Nach Filtration werden 605 g Rohgemisch erhalten.

Das Rohgemisch hat lt. gaschromatographischer Analyse folgende Zusammensetzung:
84.1 % cis-2-tertiär-Butylcyclohexanol,
4.6 % trans-2-tertiär-Butylcyclohexanol,
10.5 % cis-2-tertiär-Buthylcyclohexylacetat,
0.5 % trans-2-tertiär-Butylcyclohexylacetat und
0.2 % 2-tertiär-Butylcyclohexanon.

Das Verhältnis cis-trans 2-tertiär Butylcyclohexanol beträgt 95:5.

### Beispiele 2-13

Die Folgeansätze 2-13 verlaufen analog 1 wobei der Katalysator aus dem Startansatz 1 wieder verwendet wird. Das cis-trans Verhältnis schwankt zwischen 94:6 und 91:9.

## Patentansprüche

1. Verfahren zur Herstellung von cis-2-tertiär-Butylcyclohexanol, **dadurch gekennzeichnet, dass** 2-tertiär-Butylphenol in Gegenwart eines Nickel/Eisen Katalysatorgemisches . und 2-tertiär-Butylcyclohexylacetat hydriert wird.

2. Verfahren nach Anspruch 1, **dadurch gekennzeichnet, dass** Raney-Nickel-Eisen-Katalysatoren eingesetzt werden.

3. Verfahren nach einem der Ansprüche 1 oder 2, **dadurch gekennzeichnet, dass** die Katalysatoren im trocknen oder feuchten Zustand eingesetzt werden.

4. Verfahren nach einem der Ansprüche 1 bis 3, **dadurch gekennzeichnet, dass** die Katalysatoren im trockenen Zustand einen Eisenanteil von 2-40 Gew.-%, vorzugsweise 10-20 Gew.-%; einen Nickelanteil von 60-95 Gew.-% vorzugsweise 70-85 Gew.-% und einen Aluminiumanteil von 1-20 Gew.-% vorzugsweise 3-10 Gew.-%, aufweisen.

5. Verfahren nach einem der Ansprüche 1 bis 4, **dadurch gekennzeichnet, dass** das Gewichtsverhältnis von 2-tertiär-Butylphenol zu 2-tertiär-Butylcyclohexylacetat 100:1 - 0,2:1, vorzugsweise 7:1 - 9:1 beträgt.

6. Verfahren nach einem der Ansprüche 1 bis 5, **dadurch gekennzeichnet, dass** das Gewichtsverhältnis von Raney-Katalysator zu 2-tertiär-Butylphenol 0,0001: 1 bis 0,1:1, vorzugsweise 0,01:1 bis 0,03:1 beträgt.

7. Verfahren nach einem der Ansprüche 1 bis 6, **dadurch gekennzeichnet, dass** die Reaktionstemperatur zwischen 50 und 200°C, vorzugsweise zwischen 90 und 130°C liegt.

8. Verfahren nach einem der Ansprüche 1 bis 7, **dadurch gekennzeichnet, dass** der Wasserstoffdruck zwischen 1 und 100 bar, vorzugsweise zwischen 10 und 20 bar liegt.

9. Verfahren nach einem der Ansprüche 1 bis 8, **dadurch gekennzeichnet, dass** die Reaktionszeit zwischen 2 und 100 Stunden, vorzugsweise zwischen 5 und 20 Stunden liegt.

10. Verfahren zur Herstellung von cis-2-tertiär-Butylcyclohexylacetat, mit folgenden Schritten:
- Herstellen von cis-2-tertiär-Butylcyclohexanol nach einem Verfahren gemäß einer der Patentansprüche 1 bis 9.
- Verestern des cis-2-tertiär-Butylcyciohexanol zum cis-2-tertiär-Butylcyclohexylacetat.

## Claims

1. Method for the preparation of cis-2-tert-butylcyclohexanol, **characterised in that** 2-tert-butylphenol is hydrogenated in the presence of a nickel/iron catalyst mixture and 2-tert-butylcyclohexyl acetate.

2. Method according to Claim 1, **characterised in that** Raney nickel/iron catalysts are employed.

3. Method according to one of Claims 1 or 2, **characterised in that** the catalysts are employed in the dry or moist state.

4. Method according to one of Claims 1 to 3, **characterised in that** the catalysts in the dry state have an iron content of 2 - 40 % (m/m), preferably 10 - 20 % (m/m); a nickel content of 60 - 95 % (m/m), preferably 70 - 85 % (m/m) and an aluminium content of 1 - 20 % (m/m), preferably 3 - 10 % (m/m).

5. Method according to one of Claims 1 to 4, **characterised in that** the mass ratio of 2-tert-butylphenol to 2-tert-butylcyclohexyl acetate is 100:1 - 0.2:1, preferably 7:1 - 9:1.

6. Method according to one of Claims 1 to 5, **characterised in that** the mass ratio of Raney catalyst to 2-tert-butylphenol is 0.0001:1 to 0.1:1, preferably 0.01:1 to 0.03:1.

7. Method according to one of Claims 1 to 6, **characterised in that** the reaction temperature is between 50 and 200 °C, preferably between 90 and 130 °C.

8. Method according to one of Claims 1 to 7, **characterised in that** the hydrogen pressure is between 1 and 100 bar, preferably between 10 and 20 bar.

9. Method according to one of Claims 1 to 8, **characterised in that** the reaction time is between 2 and 100 hours, preferably between 5 and 20 hours.

10. Method for the preparation of cis-2-tert-butylcyclohexyl acetate, with the following steps:
- preparation of cis-2-tert-butylcyclohexanol by a method according to one of Patent Claims 1 to 9,
- esterification of the cis-2-tert-butylcyclohexanol to give cis-2-tert-butylcyclohexyl acetate.

## Revendications

1. Procédé de préparation du cis-2-tert-butylcyclohexanol, **caractérisé en ce que** le 2-tert-butylphénol est hydrogéné en présence d'un mélange catalytique nickel/fer et d'acétate de 2-tert-butylcyclohexyle.

2. Procédé selon la revendication 1, **caractérisé en ce que** des catalyseurs de nickel-fer de Raney sont utilisés.

3. Procédé selon l'une des revendications 1 ou 2, **caractérisé en ce que** les catalyseurs sont utilisés à l'état sec ou humide.

4. Procédé selon l'une des revendications 1 à 3, **caractérisé en ce que** les catalyseurs présentent à l'état sec une proportion de fer de 2-40 % en masse, de préférence de 10-20 % en masse, une proportion de nickel de 60-95 % en masse, de préférence de 70-85 % en masse et une proportion d'aluminium de 1-20 % en masse, de préférence de 3-10 % en masse.

5. Procédé selon l'une des revendications 1 à 4, **caractérisé en ce que** le rapport massique du 2-tert-butylphénol à l'acétate de 2-tert-butylcyclohexyle est 100:1-0,2:1, de préférence 7:1-9:1.

6. Procédé selon l'une des revendications 1 à 5, **caractérisé en ce que** le rapport massique du catalyseur de Raney au 2-tert-butylphénol est 0,0001:1 à 0,1:1, de préférence 0,01:1 à 0,03:1.

7. Procédé selon l'une des revendications 1 à 6, **caractérisé en ce que** la température de réaction est située entre 50 et 200°C, de préférence entre 90 et 130°C.

8. Procédé selon l'une des revendications 1 à 7, **caractérisé en ce que** la pression d'hydrogène est située entre 1 et 100 bar, de préférence entre 10 et 20 bar.

9. Procédé selon l'une des revendications 1 à 8, **caractérisé en ce que** la durée de réaction est située entre 2 et 100 h, de préférence entre 5 et 20 h.

10. Procédé de préparation du cis-acétate de 2-tert-butylcyclohexyle, comportant les étapes suivantes :
- préparation du cis-2-tert-butylcyclohexanol par un procédé selon l'une des revendications 1 à 9,
- estérification du cis-2-tert-butylcyclohexanol en le cis-acétate de 2-tert-butylcyclohexyle.
